# FASCICULE DE BREVET EUROPEEN

(11) **EP 2 389 235 B1**
(45) Date de publication et mention de la délivrance du brevet: **26.06.2013**
(21) Numéro de dépôt: 10706694.6
(22) Date de dépôt: 05.01.2010
(51) Int. Cl.: B01D 53/62, B01D 53/84, C02F 3/28, C02F 11/04, C12P 5/02

(54) **PROCEDE DE FIXATION DE CO2 ET DE TRAITEMENT DE DECHETS ORGANIQUES PAR COUPLAGE D'UN SYSTEME DE DIGESTION ANAEROBIE ET D'UN SYSTEME DE PRODUCTION DE MICROORGANISMES PHYTOPLANCTONIQUES**
VERFAHREN ZUR FIXIERUNG VON CO2 UND ZUR BEHANDLUNG VON ORGANISCHEM ABFALL DURCH KOPPLUNG EINES SYSTEMS ZUR ANAEROBEN FAULUNG UND EINES SYSTEMS ZUR HERSTELLUNG VON PHYTOPLANKTON-MIKROORGANISMEN
METHOD FOR THE FIXATION OF CO2 AND FOR TREATING ORGANIC WASTE BY COUPLING AN ANAEROBIC DIGESTION SYSTEM AND A PHYTOPLANKTON MICROORGANISM PRODUCTION SYSTEM

(30) Priorité: 20.01.2009 FR 0950334
(43) Date de publication de la demande: 30.11.2011
(73) Titulaire: Institut National De La Recherche Agronomique (INRA), 75007 Paris (FR); Inria Institut National de Recherche en Informatique et en Automatique, 78150 Rocquencourt (FR); INSTITUT FRANCAIS DE LA RECHERCHE POUR L'EXPLOITATION DE LA MER (IFREMER), 92130 Issy Les Moulineaux (FR)
(72) Inventeur: BERNARD, Olivier, F-06510 Carros (FR); BOUGARAN, Gael, F-44300 Nantes (FR); CADORET, Jean-Paul, F-44115 Basse Goulaine (FR); KAAS, Raymond, F-44240 La Chapelle Sur Erdre (FR); LATRILLE, Eric, F-11110 Salles D'aude (FR); SIALVE, Bruno, F-11100 Narbonne (FR); STEYER, Jean-Philippe, F-11200 Nevian (FR)
(74) Mandataire: Fourcade, Emmanuelle
(86) Numéro de dépôt international: PCT/FR2010/050008
(87) Numéro de publication internationale: WO 2010/084274

(56) Documents cités:
- EP-A2- 0 890 388
- WO-A1-91/19682
- WO-A1-2006/108532
- WO-A1-2008/099252
- US-A- 4 936 996

## Description

### Domaine de l'invention

L'invention concerne un procédé combiné de fixation de CO₂ et de traitement de déchets organiques permettant de traiter divers déchets organiques et simultanément de capter de grandes quantités de CO₂, préjudiciables pour l'environnement, provenant notamment d'effluents gazeux industriels, tout en produisant du biogaz épuré riche en méthane. Plus précisément, l'invention concerne un procédé permettant, grâce au recyclage de l'azote et d'autres nutriments provenant de la digestion anaérobie de déchets organiques, de traiter des flux importants de CO₂ annexes, au moyen de microorganismes phytoplanctoniques, tels que des microalgues et/ou des bactéries photosynthétiques. Le procédé selon l'invention permet donc de fixer du CO2 qui normalement est rejeté dans l'atmosphère, participant notamment à de l'effet de serre, et à le valoriser en bioénergie.

L'invention trouve des applications dans toute industrie générant des déchets organiques, et notamment les industries agroalimentaires, et les activités anthropiques émettrices de CO₂. L'invention trouve également des applications dans le domaine de la production de biocarburants, les biogaz épurés issus du procédé selon l'invention étant enrichis en méthane, et la biomasse notamment algale utilisée pour l'épuration des biogaz étant une source très importante de lipides également utilisables comme biocarburants.

### Etat de la technique

La méthanisation est utilisée depuis très longtemps pour la transformation des déchets organiques, solubles ou solides, en biogaz. Cette technique est appliquée à la dépollution de charges polluantes, telles que les eaux résiduaires urbaines ou industrielles chargées en matière organique biodégradable, ou les produits organiques se présentant sous forme solide, tels que les boues d'épuration, les déchets ménagers, les biodéchets, les déchets des industries agro-alimentaires, les déchets issus des différentes activités agricoles ou sylvicoles, les cultures énergétiques. La conversion de la matière organique en méthane par voie biologique offre l'avantage de fournir une énergie qui peut être utilisée directement comme carburant dans les véhicules, ou encore être convertie en chaleur et/ou électricité.

Dans un procédé de l'art antérieur représenté par le document EP-A2-0890388, des déchets organiques sont traités par fermentation impliquant une hydrolyse suivie d'une méthanisation anaérobie, le méthane produit est alors envoyé dans un premier réacteur catalytique pour former de l'hydrogène, qui sera mis en contact dans un second réacteur catalytique avec le CO₂ d'un effluent gazeux en contenant, afin de former du carbone et de l'eau.

Le traitement des charges solides par méthanisation requiert, lorsqu'il est réalisé en une seule étape, un temps particulièrement long. C'est pourquoi il est connu de procéder à un traitement en deux étapes : d'une part, l'hydrolyse au cours de laquelle les macromolécules sont transformées en molécules de faible poids moléculaire et solubles et l'acidogénèse, au cours de laquelle les molécules de faible poids moléculaire sont transformées en acides organiques à courte chaîne, en alcools, en hydrogène et autres composés simples et, d'autre part, l'acétogénèse au cours de laquelle les alcools et acides organiques sont transformés en acide acétique et la méthanogénèse, au cours de laquelle le méthane est formé, soit à partir de l'hydrogène, soit à partir de l'acide acétique.

Le biogaz produit au cours de la méthanisation est composé pour l'essentiel d'un mélange de 50 à 70 % de méthane et 30 à 50 % de dioxyde de carbone. Toutefois, en fonction des conditions opératoires du réacteur, mais aussi de la nature du ou des substrats, d'autres composés (sulfure d'hydrogène, ammoniac, siloxanes...) peuvent être présents à des concentrations plus ou moins importantes. Même à l'état de traces dans le biogaz, ces derniers peuvent se révéler délétères pour les processus de valorisation énergétique avals (corrosion des pièces mécaniques, encrassement moteurs/turbines...).

La conversion en énergie mécanique ou électrique nécessite donc dans la plupart des cas une étape préalable de filtration/épuration pour une exploitation durable.

Actuellement, de nombreuses recherches se concentrent sur la production de masse de microalgues à vocation énergétique. En effet, comme les plantes supérieures, les microalgues ont besoin pour leur croissance d'une source de carbone (inorganique - CO₂ ou HCO₃⁻- ou organique - acétate, glucose..), d'apports en éléments nutritifs (azote, phosphore, etc. mais aussi oligo-éléments, parfois vitamines, etc.) et d'énergie lumineuse. Dans des conditions de stress en éléments nutritifs, certaines espèces de microalgues sont capables d'accumuler une grande quantité de carbone sous la forme de lipides. La capacité de production lipidique de certaines espèces est bien supérieure à celle observée pour les espèces oléagineuses terrestres, rendant les microalgues particulièrement intéressantes pour les filières biocarburants.

Cependant, la production de ces microorganismes à grande échelle nécessite de mobiliser une quantité très importante d'éléments nutritifs. En effet, en considérant le rapport de Redfield (C/N/P : 106/16/1) pour une tonne de CO₂ fixée (ce qui correspond à 600 kg de biomasse sèche produite), il faut mobiliser environ 50 kg d'azote et 3.1 kg de phosphore. Sachant que pour un bassin ouvert d'un hectare de surface, il est possible de fixer une tonne de CO₂ par jour, les apports requis en azote et phosphore sont conséquents et dépassent les besoins des cultures oléagineuses terrestres.

Par ailleurs, la ressource en carbone - sous forme de CO₂ - est le facteur limitant la croissance des microalgues. Un apport continu de CO₂ au cours de la phase lumineuse de la photosynthèse permet d'atteindre des niveaux de productivité élevés en terme de biomasse et favorise aussi (dans certaines conditions) une plus grande accumulation de lipides. Cependant, l'adjonction de CO₂ dans le milieu de culture entraîne une chute du pH qui peut être délétère pour certaines espèces. Cette toxicité par une trop forte acidité nécessite le contrôle précis du flux de CO₂ en fonction du pH du milieu. Il convient donc d'asservir l'injection dans le milieu du gaz riche en CO₂ au pH de manière à maintenir le pH à une valeur de consigne.

La culture en systèmes fermés (photobioréacteurs) permet d'atteindre des niveaux de productivité très élevés en termes de fixation de CO₂ et de production de biomasse. En revanche, les coûts associés à une telle technologie (construction, fonctionnement, maintenance) rendent pour le moment cette technologie difficilement compatible avec une production de masse. A contrario, les systèmes ouverts, bien plus intéressants sur le plan économique, sont extrêmement sensibles aux contaminations de diverses natures (microalgues indigènes, bactéries, prédateurs).

De plus, les opérations de récolte des microalgues (séparation des cellules du milieu de culture) puis d'extraction des lipides de la cellule se révèlent être coûteuses en énergie et affectent considérablement le bilan énergétique de production de la biomasse. Ce poste peut en effet atteindre 50% du coût de production.

La valorisation énergétique de cette biomasse suppose une utilisation de l'ensemble de la cellule (extraction des lipides, conversion thermochimique, liquéfaction, combustion, méthanisation). L'extraction des acides gras génère par ailleurs un déchet, riche en éléments nutritifs (notamment azote et phosphore) qu'il est nécessaire de recycler.

### Exposé de l'invention

La présente invention propose de fournir un procédé de fixation de CO₂ et de traitement des déchets organiques permettant la production de bioénergie sous forme de méthane, en associant la technique de traitement de déchets par méthanisation à la technique d'épuration gazeuse par des microorganismes phytoplanctoniques tels que des microalgues et des bactéries photosynthétiques (telles que cyanobactéries), de manière à optimiser leurs avantages techniques respectifs tout en supprimant tout ou partie des inconvénients de chacune de ces techniques prises isolément.

Un but de l'invention est de fournir un système permettant le traitement de grandes quantités d'effluents gazeux, notamment industriels, par fixation du CO₂ qu'il contient.

Pour cela, l'invention propose d'associer une étape de méthanisation de déchets organiques solides à une étape de filtration du biogaz produit par des microorganismes photosynthétiques, lesdits microorganismes étant simultanément alimentés en carbone inorganique provenant d'un effluent gazeux contenant du CO₂. Afin d'éviter, ou tout le moins de réduire les risques associés d'inhibition du système selon l'invention par accumulation d'ammonium, notamment dans l'étape de méthanisation, l'invention propose de maintenir dans le digesteur de l'étape de méthanisation et/ou dans le milieu de culture de microorganismes phytoplanctoniques un rapport carbone/azote (C/N) élevé, si possible entre 10 et 35, alors qu'un tel rapport est normalement compris entre 6 et 9, ce qui conduit alors à de très fortes production d'ammonium et à de fortes inhibitions de l'étape de méthanisation. En effet, soit on agit directement sur le rapport C/N dans le digesteur, dont la phase liquide doit alimenter le méthaniseur de l'étape de méthanisation, soit on agit sur l'unité de culture de microorganismes, dont une fraction doit être réintroduite dans le digesteur, et donc indirectement dans le méthaniseur.

En effet, les microorganismes ayant vocation à croître, l'invention propose d'utiliser une partie desdits microorganismes phytoplanctoniques pour alimenter le digesteur utilisé pour l'étape de méthanisation. Selon des mises en oeuvre du procédé de l'invention, il sera possible de moduler l'apport en microorganismes et/ou en déchets organiques annexes, pouvant former un co-substrat, dans le digesteur. Ainsi, l'association d'un traitement par méthanisation à une filtration par microorganismes permet de créer une source supplémentaire de déchets organiques pour la méthanisation en utilisant l'excédent de biomasse. Par excédent, on entend une partie de la biomasse présente dans la cuve de culture de microorganismes qui peut être retirée de ladite cuve, sans que cela soit préjudiciable au bon déroulement de l'étape d'épuration du biogaz selon le procédé de l'invention. Dans la mesure où inversement l'accroissement de la biomasse peut rapidement être préjudiciable au bon fonctionnement de la cuve de culture de microorganismes, l'homme du métier trouve aisément le bon équilibre entre quantité de biomasse à maintenir dans la cuve de culture de microorganismes et quantité de biomasse excédentaire à retirer de façon à rester dans un optimum de productivité par exemple en maintenant la turbidité de façon que 95% de la lumière soit atténuée à une épaisseur entre 50% et 90% de la profondeur du bassin.

Le procédé selon l'invention peut donc utiliser des déchets organiques de nature et en quantités variables en fonction des microorganismes phytoplanctoniques présents dans le digesteur, afin d'assurer une fixation optimum du CO₂ dans l'unité de culture des microorganismes phytoplanctoniques. De même, le procédé selon l'invention peut permettre de tenir compte de la nature des déchets organiques à traiter et de la composition des microorganismes pour adapter le procédé de traitement à la nature desdits déchets organiques à traiter. Cela permet de traiter de manière homogène toutes sortes de déchets organiques.

La conversion de la matière organique lors de la méthanisation donne lieu à la production d'un biogaz comportant un mélange de méthane et de CO₂, ainsi que d'un digestat dont la fraction liquide contient des acides organiques et des éléments minéralisés (tels que de l'azote, du phosphore etc.). Le biogaz comme le digestat vont alimenter l'unité de culture de microorganismes.

L'unité de culture de microorganismes peut être un bassin ouvert permettant de solubiliser une grande quantité de CO₂. Afin de limiter les contaminations par des microorganismes extérieurs à la culture initiale, il est possible d'utiliser des espèces de microorganismes extrêmophiles, c'est-à-dire supportant des pH très élevés ou au contraire très faibles. Notamment, il est possible d'utiliser des microorganismes tels que Chlorella, Chloridella, Chlamydomonas, Viridiella, Euglena, et Euchromonoas, pour les milieux acides et Arthrospira, Nannochlorposis, Synecococcus, et Tetraselmis pour les milieux alcalins, ou une population mixte telle que celles rencontrées dans les lagunes de dépollution.

L'unité de culture comporte avantageusement deux systèmes indépendants d'apport de CO₂. L'un est destiné au CO₂ contenu dans l'effluent gazeux d'origine industrielle et l'autre au CO₂ dans le biogaz à épurer. Dans ce deuxième système, la fixation du CO₂ contenu dans le biogaz permet d'obtenir en sortie du processus un gaz à concentration élevée en méthane et au pouvoir énergétique augmenté.

L'invention a donc pour objet un procédé de fixation de CO₂ et de traitement de déchets organiques par couplage d'un système de digestion anaérobie et d'un système de production de microorganismes phytoplanctoniques, comportant les étapes suivantes :
(a') on traite des microorganismes (105) issus d'une culture phytoplanctonique et des déchets organiques (104) dans un réacteur d'hydrolyse (101);
(a") on traite au moins une partie d'un effluent liquide (109) sortant de l'étape (a') dans un réacteur de méthanisation (102) ;
(b) on traite une phase liquide (127) et du biogaz à épurer (110) sortant de l'étape (a") dans une unité de culture de microorganismes phytoplanctoniques (103) ;
(c) on injecte un effluent gazeux (113) contenant du CO₂ dans l'unité de culture de microorganismes phytoplanctoniques ;
(d) on maintient une concentration en NH₃ inférieure à 0,5 g/L dans le réacteur de méthanisation ;
(e) on récupère un biogaz enrichi en méthane au sortir de l'unité de culture de microorganismes phytoplanctoniques.

Le biogaz à épurer récupéré au sortir de l'étape (a") s'entend d'un gaz produit par la fermentation de matières organiques, d'origine animale et/ou végétale, en l'absence d'oxygène. Le biogaz est composé essentiellement de méthane, et de gaz carbonique avec éventuellement de faibles quantités d'eau, de sulfure d'hydrogène etc.

Le biogaz épuré, enrichi en méthane, récupéré au sortir de l'étape (e) comporte essentiellement du méthane et a une teneur limitée en oxygène. Il est également possible d'avoir d'autres gaz, tels que le: CO₂, et/ou le N₂ mais à l'état de traces. D'une manière générale, le biogaz épuré enrichi en méthane récupéré au sortir de l'étape (e) comporte au moins 90% de méthane.

La méthanisation en deux étapes (a') et (a") permet de placer les populations hydrolytiques/acidogènes et méthanogènes dans leurs conditions optimales respectives, d'atteindre des rendements de conversion de la matière organique plus élevés, et de pouvoir traiter un plus large spectre de déchets organiques du fait de l'effet tampon du réacteur d'hydrolyse. Par ailleurs, le biogaz produit au niveau de l'étape de méthanisation a une concentration en méthane particulièrement élevée, et le CO₂ produit lors l'étape d'hydrolyse (a') peut avantageusement être injecté dans la culture de microalgues, au même titre que le biogaz produit au niveau de l'étape de méthanisation proprement dite (a").

Bien entendu, il est possible, pour des raisons d'encombrement ou autre, de réaliser de manière connue la méthanisation en une unique étape (a), le biogaz à épurer étant récupéré au sortir du réacteur unique.

L'étape de méthanisation, du fait de la consommation des acides organiques, tend à augmenter le pH dans le méthaniseur, ou réacteur de méthanisation, entraînant une augmentation du rapport NH₃/NH₄⁺ dans ledit méthaniseur. Or, une trop forte concentration en NH₃ dans le méthaniseur risquerait à court terme d'inhiber le procédé selon l'invention.

Aussi, selon l'invention, pour maintenir dans le méthaniseur une concentration inférieure à 0,5 g/L de NH₃ on propose de maintenir un rapport Carbone/Azote (C/N) moyen entre 10 et 35 dans le réacteur d'hydrolyse et/ou dans l'unité de culture de microorganismes et/ou d'injecter directement dans le méthaniseur du CO₂ afin de limiter la remontée du pH.

Par rapport C/N moyen, on entend la moyenne des rapports C/N dans les déchets, les cellules et dans le milieu de culture, les microorganismes pouvant excréter une grande quantité de carbone sous forme de polymères organiques. Le maintien d'un rapport C/N compris entre 10 et 35, qui est la conséquence d'un fort apport en CO₂ dans l'unité de culture de microorganismes phytoplanctoniques permet d'éviter l'inhibition du procédé selon l'invention par accumulation d'ammonium..

Pour cela, selon l'invention, on peut par exemple moduler en qualité et/ou en quantité la fraction de déchets organiques introduite dans le réacteur d'hydrolyse à l'étape (a'). Notamment, on peut prévoir d'utiliser des déchets organiques présentant un rapport C/N supérieur à 25.

Autrement, il est possible de moduler la quantité d'effluent liquide sortant de l'étape (a") introduite dans l'étape (b), de manière à induire une limitation nutritive propre à modifier la composition des microorganismes de l'unité de culture de microorganismes phytoplanctoniques pour favoriser l'accumulation de lipides et glucides dans lesdits microorganismes, et donc l'augmentation du rapport C/N dans ladite unité.

Il est aussi possible de moduler le débit entrant du biogaz sortant de l'étape (a") dans l'unité de culture de microorganismes phytoplanctoniques, de manière à contrôler le pH de ladite unité de culture et créer des conditions propres à augmenter le rapport C/N.

En effet, la qualité du contenu intracellulaire des microalgues est, dans une certaine mesure, dépendante de leurs conditions de culture. Ainsi, en jouant sur les conditions opératoires appliquées lors de la culture des microorganismes, il est possible de modifier notablement la répartition des compartiments protéinique, lipidique et glucidique de la matière organique. Ces modifications ont une influence sur la biodégradabilité des cellules et leur conversion en acides organiques et en méthane. Les variations entre les fractions azotées (protéines) et carbonées (glucides et lipides) de la matière organique affectent le rapport carbone/azote global (C/N) apporté dans le réacteur d'hydrolyse et donc par conséquent également dans l'unité de culture de microorganismes phytoplanctoniques.

Selon l'invention, le contrôle des conditions de culture des microorganismes peut ainsi se faire par modulation de l'apport de CO₂ et/ou par modulation de l'apport d'éléments nutritifs contenus dans la phase liquide récupérée au sortir des étapes (a') et/ou (a"). Le CO₂ provient dans tous les cas d'un effluent gazeux, contenant une forte teneur en CO₂ (entre 5 et 25%) mais peut également provenir du réacteur d'hydrolyse où a eu lieu la première étape de traitement des déchets organiques. Plus précisément, selon l'invention, il est possible de prévoir une étape lors de laquelle on module la quantité d'effluent liquide sortant de l'étape (a") introduite dans l'unité de culture de microorganismes phytoplanctoniques, et/ou la quantité de CO₂ organique et/ou inorganique introduite dans ladite unité de culture de microorganismes, de manière à modifier la composition de la biomasse de l'unité de culture de microorganismes et maintenir un rapport C/N entre 10 et 35.

Le contrôle de ces deux paramètres (nutriments et CO₂) permet de modifier la composition des cellules, et ainsi le rapport C/N. La croissance rapide des microorganismes (environ un doublement de la population par jour) laisse envisager une réponse rapide des cellules.

Dans d'autres conditions, les qualités et quantités de co-déchets peuvent être ajustées pour s'adapter à la qualité des microorganismes afin de rester dans les conditions optimales de dégradabilité.

Cette étape permet, en augmentant le flux d'entrée de la phase liquide dans l'unité de culture de microorganismes, de favoriser la croissance de la biomasse, car on augmente les apports en nutriments et en azote. En effet, cette phase liquide contient en plus des acides organiques, essentiellement de l'azote et du phosphore sous forme minérale (ammonium et phosphate). Ces derniers éléments sont nécessaires au métabolisme des microorganismes phytoplanctoniques tels que les microalgues. Certains acides organiques sont assimilables par certaines espèces de microalgues (croissance hétérotrophe ou mixotrophe) et permettent d'augmenter significativement la croissance.

A l'inverse, en diminuant le flux d'entrée de la phase liquide dans l'unité de culture de microorganismes, on augmente le rapport C/N dans ladite unité de culture. En effet, les carences en éléments nutritifs ont un impact sur la physiologie des cellules de microalgues. La limitation ou la carence en azote favorise l'accumulation de carbone dans la cellule sous forme de glucides, d'amidon ou de lipides. Cependant, ce phénomène s'accompagne d'un ralentissement de la croissance, faute du précurseur azote nécessaire à l'élaboration des protéines. Il est donc nécessaire de contrôler le flux d'entrée d'effluent liquide de manière à ne pas entraîner un arrêt total préjudiciable de la croissance des microorganismes.

De même, en modulant le débit de biogaz sortant de l'étape (a") injecté dans l'unité de culture de microorganismes, on peut modifier la composition de la biomasse de l'unité de culture de microorganismes phytoplanctoniques (mais également leur productivité) en fonction des besoins en carbone et azote. En augmentant la quantité de biogaz dans l'unité de culture de microorganismes phytoplanctoniques, on favorise l'accumulation d'amidon et de lipides dans les cellules, ce qui permet d'augmenter le rapport C/N dans les microorganismes.

De même, le débit d'entrée de l'effluent gazeux contenant du CO₂ peut être modulé, l'apport de CO₂ permettant de modifier la composition de la biomasse de l'unité de culture de microorganismes. Si la culture est à pH alcalin le CO₂ se dissoudra spontanément. Que la culture soit de type acide ou alcalin, l'ajout de gaz sera conditionné par le pH via un système de régulation. L'injection dans le milieu du gaz riche en CO₂ est avantageusement asservie au pH de manière à maintenir le pH à une valeur de consigne. Des régulateurs type PID ou MLI peuvent être utilisés pour réaliser cette tâche. Si l'alcalinité est élevée (présence de grandes quantités de cations), le pH peut être maintenu à des valeurs élevées malgré les flux de CO₂.

On peut également moduler le débit d'entrée d'effluent gazeux de manière à maintenir un flux de carbone fixé au moins 10 fois supérieur au flux d'azote entrant dans l'unité de culture de microorganismes. Pour cela, on peut imposer un taux de dilution de la culture de microorganismes phytoplanctoniques inférieur au taux de croissance maximum desdits microorganismes, de manière à induire une limitation par un élément nutritif, le carbone inorganique étant lui apporté en excès.

Par ailleurs, l'ajout de CO₂, de préférence piloté par la valeur du pH, dans l'unité de culture de microorganismes, permet de maintenir le CO₂ dissous dans un domaine non limitant pour la photosynthèse. Cela permet d'éviter un phénomène classique de montée du pH dans les cultures à forte densité, associé à une raréfaction du carbone inorganique dissous nécessaire à la croissance. L'injection de CO₂ permet donc dans le cas de cultures acidophiles et dans le cas de cultures alcalines de maintenir des taux de croissance élevés, y compris pour des concentrations en biomasses élevées dans la culture en maintenant un pH légèrement acide (entre 4 et 7). Seules certaines microalgues ont la capacité de se développer dans ces gammes de pH, ce qui limite la biodiversité qui devient, comme cela a été observé pour des écosystèmes naturels, plus simple dans sa composition.

De plus, l'addition de CO₂ augmente de façon significative la concentration en biomasse, ce qui induit une augmentation des performances épuratoires de l'azote et du phosphore.

Il est également possible, pour maintenir un rapport C/N moyen dans l'unité de culture de microorganismes phytoplanctoniques entre 10 et 35, d'utiliser des déchets organiques présentant un rapport C/N supérieur à 25.

De même, il est possible d'utiliser comme microorganismes pour l'étape (b) des espèces autotrophes, ne consommant que du carbone inorganique, ayant un rapport C/N supérieur à 10. (par exemple Staurastrum sp.).

Tout ou parties de ces solutions pour maintenir un rapport C/N moyen entre 10 et 35 dans le réacteur d'hydrolyse comme dans l'unité de culture de microorganismes peuvent être cumulées pour permettre le maintien dudit rapport dans les proportions souhaitées et ainsi une concentration en NH3 inférieure à 0.5 g/L dans le méthaniseur.

Autrement, comme évoqué plus haut, pour maintenir une concentration en NH3 inférieure à 0,5 g/L dans le méthaniseur, on prévoit une étape supplémentaire (f) lors de laquelle un effluent gazeux contenant du CO₂ est introduit dans le réacteur de méthanisation de l'étape (a"), afin d'y maintenir un pH d'environ 7,5, et plus généralement compris entre 7 et 8, pour éviter une inhibition de la méthanogénèse par accumulation d'ammoniac. En effet, l'effet principal de la production d'ammonium est une alcalinisation du milieu qui conduit à une augmentation du pH favorisant alors la forme NH3 (toxique) au détriment de la forme NH4⁺. L'injection de CO₂ dans le digesteur, grâce à une pompe adhoc, est asservie au pH du réacteur de méthanisation, de manière à maintenir le pH aux alentours de 7,5. A noter que cet effet « naturel » de production d'alcalinité évite le recours à des produits chimiques alcalins tels que soude ou bicarbonate de potassium qui grèvent les coûts des dispositifs de dépollution.

De plus, il est possible, lorsque le pH en sortie de l'étape a' est naturellement alcalin, selon une étape supplémentaire (g) de filtrer le biogaz issu de l'étape (a") sur une colonne d'échange dans laquelle le biogaz est injecté par le bas. Lors de la remontée des bulles, le CO₂ se dissout et passe sous forme de bicarbonate, alors que le méthane à faible solubilité est récupéré à la surface du réacteur avant l'étape (b), de manière à profiter de l'alcalinité induite par l'ammonium pour dissoudre le CO₂ et récupérer le méthane épuré. La conséquence est également de limiter la basification du milieu dans le réacteur de méthanisation, et donc de réduire la toxicité de l'ammonium: Le biogaz épuré lors de cette étape intermédiaire (f) contient encore du CO₂ et est donc avantageusement injecté dans l'unité de culture de microorganismes phytoplanctoniques pour qu'il y soit entièrement fixé.

Il est également possible, selon le procédé de l'invention, d'injecter une fraction de l'effluent liquide sortant de l'étape (a') directement dans l'unité de culture de microorganismes phytoplanctoniques. Une telle injection se déroule préférentiellement la nuit pour ne pas entrer en concurrence avec la photosynthèse et assurer la production de la biomasse de microalgues y compris lors de la phase nocturne.

Avantageusement, on utilise dans l'unité de culture de microorganismes phytoplanctoniques des espèces acidophiles ou basophiles pour limiter les contaminations dans ladite unité.

Avantageusement, on stocke provisoirement le biogaz récupéré au sortir de l'étape (a") dans un réservoir tampon avant de l'introduire dans l'unité de culture de microorganismes. L'utilisation d'un tel réservoir permet de moduler aisément l'apport en biogaz dans la cuve de culture de microorganismes en fonction des besoins et de stocker le biogaz pendant la phase nocturne.

Le procédé selon l'invention peut par ailleurs permettre de produire un biocarburant supplémentaire, en plus du biogaz épuré enrichi en méthane, en extrayant et récupérant des composés valorisables, tels que des lipides, de la biomasse de microorganismes, avant d'injecter le résidu, c'est à dire la biomasse sans composé valorisable, dans le réacteur d'hydrolyse.

Préférentiellement, on concentre la biomasse provenant de l'unité de culture de microorganismes, de manière à séparer le surnageant de ladite biomasse, avant d'introduire le concentrat de biomasse dans le réacteur d'hydrolyse, la phase liquide pouvant elle être réintroduite dans l'unité de culture de microorganismes.

L'invention concerne également un système couplé de traitement de déchets organiques et de fixation de CO₂, apte à mettre en oeuvre le procédé selon l'invention, comportant au moins
- un réacteur d'hydrolyse/acidogénèse couplé à un réacteur de méthanisation,
- une unité de culture de microorganismes phytoplanctoniques,
- un premier conduit d'alimentation apte à acheminer un biogaz à épurer depuis le réacteur de méthanisation jusqu'à l'unité de culture de microorganismes phytoplanctoniques,
- un second conduit d'alimentation apte à acheminer une phase liquide riche en nutriments depuis le réacteur d'hydrolyse et/ou le réacteur de méthanisation jusqu'à l'unité de culture de microorganismes phytoplanctoniques,
- un troisième conduit d'alimentation apte à acheminer un effluent gazeux extérieur audit système et contenant du CO₂ jusqu'à l'unité de culture de microorganismes phytoplanctoniques et
- un conduit d'évacuation et de récupération de biogaz épuré enrichi en méthane au sortir de l'unité de culture de microorganismes phytoplanctoniques.

### Description détaillée des figures et de l'invention

L'invention sera mieux comprise à la lecture de la description qui suit et à l'examen des figures qui l'accompagnent. Celles-ci sont présentées à titre indicatif et nullement limitatif de l'invention. Les figures représentent :
Figure 1 : une représentation schématique d'une installation selon un mode de réalisation, apte à mettre en oeuvre le procédé selon l'invention ;
Figure 2 : un agrandissement du dispositif d'épuration du biogaz contenu dans l'unité de culture de microorganismes phytoplanctoniques de la figure 1.

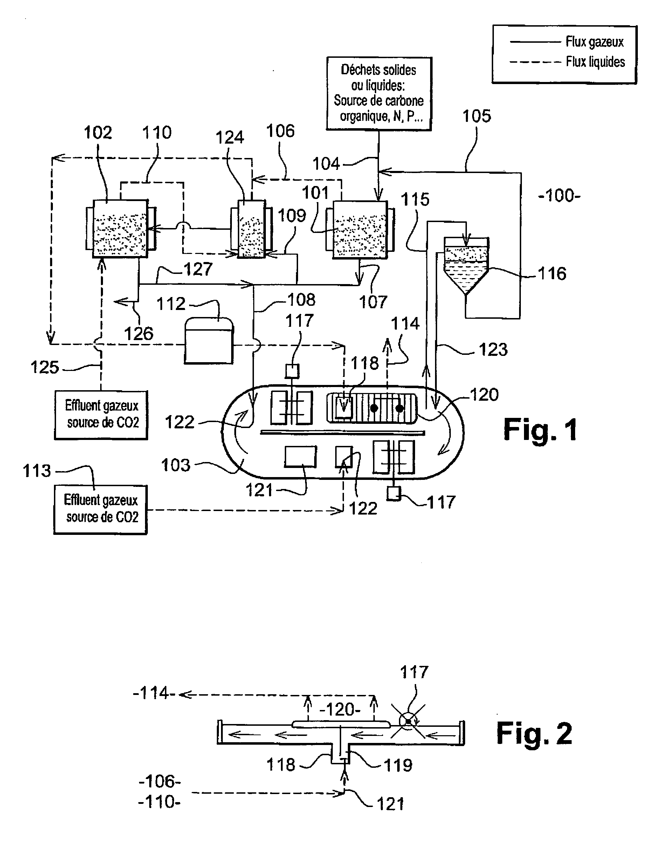

Dans l'exemple représenté à la figure 1, un système de traitement de déchets organiques et de fixation de CO₂ 100 mettant en oeuvre le procédé selon l'invention comporte trois unités principales, respectivement réacteur d'hydrolyse/acidogénèse 101, réacteur de méthanisation 102 et unité de culture de microorganismes phytoplanctoniques 103.

L'unité de culture de microorganismes phytoplanctoniques 103 est par exemple un bassin ouvert de faible profondeur (20 à 50 cm). L'agitation est assurée de manière connue par deux roues à aubes 117 qui permettent une recirculation et un mélange du milieu de culture et des microorganismes.

La matière organique provenant de déchets organiques 104 d'une part et, des cultures de microorganismes et/ou de résidus de microorganismes 105, après extraction ou non de composés d'intérêt, d'autre part, est injectée grâce à des conduits d'alimentation dans le réacteur d'hydrolyse/acidogénèse 101 au sein duquel des organismes hydrolytiques et acidogènes (Clostridium Bacillus Escherichia Staphylococcus, etc) la convertissent en acides organiques et molécules hydrolysées.

Les éléments contenus dans la matière organique, tels que l'azote et le phosphore, sont également minéralisés.

Le gaz 106 produit est composé pour l'essentiel de dioxyde de carbone et dans une moindre mesure d'hydrogène.

Une fraction 108 de la sortie liquide 107 du réacteur d'hydrolyse/acidogénèse 101, dont le pH est généralement acide (autour de 5 ou 6), est introduite par un conduit d'alimentation spécifique dans l'unité de culture de microorganismes phytoplanctoniques 103. Cela permet d'augmenter la production de biomasse, de favoriser la croissance de la biomasse en l'absence de lumière (donc la nuit), et permet l'accumulation de lipides en carence azotée.

Le reste 109 de la sortie liquide 107 est introduit dans un réacteur de filtration du biogaz 124, avant d'être introduit dans le réacteur de méthanisation 102. Ce réacteur de filtration 124 est utilisé dans le cas où le rapport C/N faible (entre 5 et 20) en entrée du réacteur d'hydrolyse/acidogénèse 101 conduit à une forte libération d'ammonium et donc à des pH supérieurs à 8. L'alcalinité de la sortie liquide 107 est alors utilisée pour dissoudre le CO₂, alors que le méthane, peu soluble est évacué par le haut du méthaniseur.

La population méthanogène (methanosarcina, methanococcus, methanobacterium, etc) du réacteur anaérobie de méthanisation 102 convertit ces acides en un biogaz 110 essentiellement composé de méthane et de dioxyde de carbone.

Avantageusement, le biogaz 110 transite également par le réacteur de filtration 124 avant d'être injecté par un conduit d'alimentation dans l'unité de culture de microorganismes phytoplanctoniques 103. Il y a ainsi une première filtration du biogaz. En effet, l'alcalinité induite par l'ammonium permet de dissoudre une partie du CO₂ contenu dans le biogaz à épurer et de récupérer le méthane partiellement épuré. Cela permet par ailleurs de limiter la basification du milieu dans le réacteur de méthanisation 102 et donc de réduire la toxicité de l'ammonium.

Une partie du digestat 126, ou phase liquide, issu du processus de méthanisation peut également être destinée à une valorisation agronomique au sortir du réacteur de méthanisation 102. Le reste 127 est introduit dans l'unité de culture de microorganismes 103, avec la fraction d'effluent liquide 108 provenant du réacteur d'hydrolyse 101.

Les microorganismes présents dans l'unité de culture 103 utilisent pour leurs besoins nutritifs des éléments tels que NH₄⁺ et PO₄³⁻ issus du réacteur d'hydrolyse/acidogénèse 101 et comme source de carbone, le CO₂ présent dans les gaz 106, 110 récupérés au sortir des réacteurs d'hydrolyse/acidogénèse 101 et de méthanisation 102 et acheminés au moyen de conduits d'alimentation jusqu'à l'unité de culture de microorganismes 103. Ces gaz 106, 110 sont avantageusement stockés dans un réservoir 112 avant d'être injecté dans l'unité de culture de microorganismes 103. Le gaz 106, 110 provenant des réacteur d'hydrolyse 101 et de méthanisation 102 comporte, en plus du méthane, du CO₂, mais peut également contenir du souffre et/ou d'autres composants préjudiciables à une utilisation directe du biogaz, et doit donc être épuré.

Plus précisément, les gaz 106, 110 issus des réacteurs d'hydrolyse/acidogénèse 101 et de méthanisation 102 sont injectés dans le bassin 121 de l'unité de culture de microorganismes 103, grâce à un dispositif d'épuration 118 (voir agrandissement figure 2) bien connu de l'homme du métier, favorisant à la fois le transfert du flux gazeux 119 et la récupération du gaz dans une cloche de récupération 120 après passage dans l'unité de culture 103.

Le dispositif d'épuration 118 comporte un bassin 121 muni de plusieurs puits de 100 à 150 cm de profondeur, au fond desquels se trouvent des diffuseurs. Le transfert se réalise par l'intermédiaire de ces puits qui génèrent de fines bulles de gaz, afin d'assurer un transfert optimal vers la phase liquide, et cette colonne de bulles est traversée par le milieu de culture. Lorsque la culture de microorganismes arrive au niveau des diffuseurs, elle a consommé l'essentiel du stock de carbone inorganique dissous, et de ce fait le pH est remonté à des valeurs plus élevées. Le CO₂ contenu dans le gaz est ainsi rapidement transféré dans le milieu de culture où il est stocké principalement sous forme de bicarbonate. Le CO₂ non fixé, ainsi que les composés gazeux, à savoir le méthane et l'hydrogène, qui ne sont pas transférés vers la phase liquide, sont récupérés en surface du bassin 121, en assurant au besoin une légère dépressurisation.

L'apport permanent de CO₂ permet de maintenir en certains points du bassin 121, le jour, un pH régulé à une valeur acide (en dessous de 6,5) ou basique (au dessus de 8.5), limitant ainsi le développement de microorganismes non souhaités.

En effet, la solubilisation du CO₂ (sous forme de bicarbonate et de CO₂), implique un contrôle du pH aux points d'injection 122 du gaz afin de veiller à ce que les microorganismes ne soient jamais limités en carbone inorganique (pH inférieur à 8,5 pour les espèces non alcalinophiles), que le milieu au point d'injection 122 du gaz ait une concentration faible en carbone inorganique pour un transfert rapide du CO₂ vers la phase liquide, alors que le méthane reste principalement sous forme gazeuse celui-ci ayant une solubilité très inférieure, et maintenir des conditions acides locales.

Par ailleurs, le point d'injection 122 du gaz étant situé à proximité des roues à aubes 117, l'oxygène dissous est massivement dégazé, ce qui limite fortement sa présence dans le biogaz récupéré et épuré de son CO₂. Dès lors, au point d'injection 122, la valeur de pH du milieu est supérieure, le CO₂ ayant été consommé, et la concentration en oxygène dissous est très faible.

Les valeurs de consigne du pH aux points d'injection 122 sont calculées de manière à s'assurer que le milieu de culture qui arrive au point d'injection 122 a une capacité de stockage de CO₂ suffisant. De la sorte, la grande majorité du CO₂ est absorbée, alors que le méthane traverse la phase liquide pour se retrouver beaucoup plus concentré dans le gaz (voir exemple de calcul des pH ci-dessous).

L'opération de filtration du biogaz à épurer 110 par les microorganismes phytoplanctoniques de l'unité de culture 103 permet de produire en sortie 114 un biogaz épuré, à forte teneur en méthane, qui peut être valorisé énergétiquement ou stocké pour un usage ultérieur.

Un effluent gazeux 113, provenant par exemple d'une activité anthropique, est également utilisé comme source de carbone pour alimenter l'unité de culture de microalgues 103. Cet effluent gazeux 113 est acheminé par un conduit d'alimentation spécifique depuis la source, un réservoir par exemple, jusqu'à l'unité de culture de microorganismes phytoplanctoniques.

Le même dispositif de filtration 118 est utilisé pour la fixation du CO₂ contenu dans l'effluent gazeux 113, à la différence que celui-ci ne dispose pas nécessairement de la cloche de récupération du gaz 120.

L'ajout de l'effluent gazeux source de CO₂ 113 est conditionné par le pH via un système de régulation. Le débit de gaz riche en CO₂ est régulé en fonction du pH en aval du point d'injection de manière à maintenir ce pH à une valeur de consigne. Un régulateur de type PID, RST ou MLI est avantageusement utilisé. L'espacement entre la sonde pH et le point d'injection du gaz doit correspondre à la distance parcourue par le fluide en mouvement entre deux instants d'injection. Par exemple, si la vitesse du fluide est de 30 cm/s, et que le calcul de l'injection par le système de régulation est lancé toutes les 3 secondes, la sonde doit se situer à 90cm du point d'injection.

La biomasse algale et bactérienne en excès 115 est retirée de l'unité de culture de microalgues 103. Pour une meilleure efficacité du système, l'exportation de biomasse est gérée de manière à assurer un fonctionnement proche de l'optimum de productivité du système (qui est variable selon la lumière incidente) un fonctionnement en turbidostat permettant d'asservir la concentration en fonction de la lumière incidente et de s'assurer que la culture est toujours en nutriment limitant. La biomasse en excès 115 est dirigée vers un décanteur 116 et le décantât 105, qui a éventuellement subi une extraction des composés valorisés, est acheminé par conduit vers le réacteur d'hydrolyse/acidogénèse 101.

Selon des utilisations du système de traitement 100, le décantât 105 peut être utilisé comme seul substrat dans le réacteur d'hydrolyse/acidogénèse 101, ou en mélange pour être co-digéré avec des déchets organiques 104. Le surnageant 123 est, au besoin, réutilisé dans l'unité de culture des microalgues 103.

Une source de CO₂ extérieur 125, sous forme d'un effluent gazeux, par exemple industriel, contenant du CO₂ peut être introduit dans le réacteur de méthanisation 102, afin d'y maintenir un pH autour de 7,5. Le gaz 110 au sortir du réacteur de méthanisation 102 est alors très chargé en carbone inorganique.

Le contrôle de la qualité de la biomasse de l'unité de culture de microorganismes phytoplanctoniques 103 permet de l'améliorer en augmentant la concentration en lipide ou en amidon intracellulaire en fonction des besoins, c'est-à-dire en fonction du rapport C/N a maintenir entre 10 et 35, mais également si on souhaite optimiser le traitement des déchets organiques en fonction de la nature des déchets organiques 104 à traiter dans le réacteur d'hydrolyse 101. Cela permet également d'augmenter la quantité de biogaz épuré 114 produite par unité de masse de microorganismes.

Avant injection dans le réacteur d'hydrolyse 101, les microorganismes 105 peuvent être soumis à un pré-traitement physique et/ou chimique (thermique, acido-basique, ozonation...) afin d'en améliorer la digestibilité et d'augmenter ainsi la productivité.

La biomasse recyclée 105 peut être associée à un autre substrat, avec ou sans prétraitement.

### Exemples

### 1.1 Exemple de calcul des pH à appliquer dans l'unité de culture de microorganismes

L'exemple est donné pour une culture en bassin ouvert de 150 m de long dont le taux de fixation instantané de CO₂ est de 2 mmol/l/h. Ce sont les valeurs moyennes classiques pour ce type d'unité de culture de microorganismes, correspondant par exemple à une biomasse de 0.4 g/l et un taux de croissance instantané de 2,3 jour⁻¹.

Cela induit des augmentations de pH, en fonction de l'alcalinité du milieu, de l'ordre de 0,1 à 0,5 unités pH par minute.

Pour une culture s'écoulant à 30 cm.s⁻¹, les 75 mètres séparant les deux diffuseurs sont atteints en 4,2 minutes, ce qui correspond à une augmentation du pH de 0,4 à 2,1 unités pH. Cette augmentation du pH est renforcée après le passage par les roues à aubes qui permet le dégazage d'une partie du CO₂.

Il faut alors fixer la consigne du pH au point d'injection du gaz à une valeur entre 5 et 6,5. Pour une boucle de contrôle lancée toutes les 3 secondes, il faut placer la sonde pH à 90cm en aval du point d'injection.

### 1.2 Exemple de dimensionnement

Comme les taux de dilution maximum respectifs du procédé de digestion anaérobie et de la culture phototrophe sont du même ordre de grandeur, le rapport des volumes entre le bassin où sont cultivés les microorganismes et le méthaniseur correspondra au facteur de concentration des microorganismes après prélèvement. Par exemple, si les microorganismes ont pu être concentrés d'un facteur 100, le débit associé à traiter sera 100 fois plus faible, et le volume du méthaniseur sera donc 100 fois plus faible. Ainsi on peut imaginer passer de 0.5g/l de matière sèche dans le bassin à 50 g/l dans le méthaniseur avec un débit 100 fois plus faible. La valeur de 50g/l en entrée du méthaniseur correspond à une valeur de 25g/l de carbone, soit 1 à 4 g/l d'azote qui est donc un maximum à ne pas dépasser.

Pour une profondeur de bassin de 10 cm, cela correspond à un digesteur 1000 m³ par hectare de bassin.

Le bassin aura alors une capacité de traitement de 0.5 g/l/jour, soit 0.25 gC/l/jour, ou encore 0.9 gCO₂/l/jour, soit 900 kg de CO₂ par hectare. La production associée aux 500kg de matière sèche par hectare et par jour est de 600kg de DCO par hectare, ce qui correspond à 220 m³ de méthane (et 95 m³ de CO₂, soit 185 kilo de CO₂). Ceci correspond à un contenu énergétique de 1800 kWh. Par ailleurs le flux d'azote par jour et par hectare est de 10 à 40 kilogrammes, qu'il aurait fallu apporter par des engrais sans le recyclage de l'azote via la digestion anaérobie. L'apport de l'étape de la méthanisation sera de l'ordre de 370 kilogrammes de carbone inorganique.

### 1.3 Exemple de mise en oeuvre du procédé

En se calant sur les calculs qui précèdent, pour un bassin de un hectare :
- nature des déchets organiques et quantités : type vinasses ; 1m3/jour à 50kg/m³ (C/N=200).
- quantités d'effluent liquide 108 et 127 introduites dans l'unité de culture de microorganismes 103 : respectivement 0 m³/jour et 9 m³/jour
- nature des microorganismes utilisés : microalgues acidophiles type chlamydomonas acidophila maintenues à pH 4 (C/N=6)
- quantité 105 de microorganismes prélevée dans l'unité de culture et introduite dans le réacteur d'hydrolyse 101 : 500 kg/jour, soit 9 m³ et 104 1 m³/jour, soit 50kg, le rapport C/N moyen étant alors de 25.
- pH aux points d'injection 122 dans le bassin 118 : pH 4
- quantité d'effluent industriel contenant du CO₂ 113 introduite dans l'unité de culture de microorganismes 103: 715 kg de CO₂ fixé, ce qui correspond à une injection (pour une efficacité de transfert de 20%) de 3.5 tonnes de CO₂ industriel.
- quantité d'effluent industriel contenant du CO₂ 125 introduite dans le réacteur de méthanisation 102 : 0 car C/N maintenu entre 10 et 35 dans l'unité de culture de microalgues.
- quantité de biogaz épuré enrichi en méthane récupéré au sortir de l'unité de culture de microorganismes : 220 m³/jour
- temps de fonctionnement du système : Nettoyage du système tous les 6 mois.

### BIBLIOGRAPHIE

Angelidaki I, Ahring BK. Thermophilic anaerobic digestion of livestock waste : the effect of ammonia. Appl. Microbiol. Biotechnol. 1993;38:560-564.
Angelidaki I, Ellegaard L, Ahring BK. Applications of the anaerobic digestion process. In: Ahring BK, editor. Biomethanation, Springer Berlin /Heidelberg, 2003. p.1-33.
Braun R, Huber P, Meyrath J. Ammonia toxicity in liquid piggery manure digestion. Biotechnol. Lett. 1981;3:159-164.
Chen PH. Factors influencing methane fermentation of micro-algae. PhD thesis, University of California, Berkeley, CA, USA, 1987.
Chen Y, Cheng JJ, Creamer KS. Inhibition of anaerobic digestion process: A review. Bioresour. Technol. 2008;99:4044-4064.
Chisti Y. Biodiesel from microalgae. Biotechnol. Adv. 2007;25: 294-306.
Cime DG, Paloumet X, Björnsson L, Alves MM, Mattiasson B. Anaerobic digestion of lipid-rich waste--Effects of lipid concentration. Renew. Energy. 2007 ;32 :965-975.
Doucha J, Straka F, Livansky K. Utilization of flue gas for cultivation of microalgae (Chlorella sp.) in an outdoor open thin-layer photobioreactor. J. Appl. Phycol. 2005;17:403-412.
Golueke CG, Oswald WJ, Gotaas HB. Anaerobic digestion of algae. Appl. Microbiol. 1957;5:47-55.
Grobbelaar JU. Algal Nutrition. In Richmond A, editor. Handbook of microalgal culture: biotechnology and applied phycology, Wiley-Blackwell, 1984.
Heubeck S, Craggs RJ, Shilton A. Influence of CO2 scrubbing from biogas on the treatment performance of a high rate algal pond. Water Sci. Technol. 2007;55(11):193-200.
Koster IW, Lettinga G. The influence of ammonium-nitrogen on the specific activity of pellitized methanogenic sludge. Agric. Wastes. 1984;9:205-216.
Koster IW, Lettinga G. Anaerobic digestion at extreme ammonia concentrations. Biol. Wastes 1988;25:51-59.
Maeda K, Owada M, Kimura N, Omata K, Karube I. CO2 fixation from the flue gas on coal-fired thermal power plant by microalgae. Energy Conv. Manag. 1995.36:717-720.
Mandeno G, Craggs R, Tanner C, Suskias J, Webster-Brown J. Potential biogas scrubbing using a high rate pond. Water Sci. Technol. 2005;51 (12):253-256.
Munoz R, Jacinto M, Guieysse B, Mattiasson B. Combined carbon and nitrogen removal from acetonitrile using algal-bacterial bioreactors. Appl. Microbiol. Biotechnol. 2005;67:699-707.
Olaizola M. Commercial development of microalgal biotechnology: from the test tube to the marketplace. Biomol. Eng. 2003;20:459-466.
Omil F, Mendez R, Lema JM. Anaerobic treatment of saline wastewaters under high sulfide and ammonia content. Bioresour. Technol. 1995;54:269-278.
Oswald WJ, Golueke CG. Biological transformation of solar energy. Adv. Appl. Microbiol. 1960;2:223-262.
Samson R, LeDuy A. Biogas production from anaerobic digestion of Spirulina maxima algal biomass. Biotechnol. Bioeng. 1982;24:1919-1924.
Samson R, LeDuy A. Detailed study of anaerobic digestion of Spirulina maxima algae biomass. Biotechnol. Bioeng. 1986;28:1014-1023.
Sanchez EP, Travieso L. Anaerobic digestion of Chlorella vulgaris for energy production." Resour, Conserv. Recycl. 1993;9:127-132.
Travieso L, Sanchez EP, Benitez F, Conde JL. Arthospira sp. intensive cultures for food and biogas purification. Biotechnol. Lett. 1993;15:1091-1094.
Yen HW, Brune DE. Anaerobic co-digestion of algal sludge and waste paper to produce methane. Bioresour. Technol. 2007;98:130-134.

## Revendications

1. Procédé de fixation de CO₂ et de traitement de déchets organiques par couplage d'un système de digestion anaérobie et d'un système de production de microorganismes phytoplanctoniques, comportant les étapes suivantes :
(a') on traite des microorganismes (105) issus d'une culture phytoplanctonique et des déchets organiques (104) dans un réacteur d'hydrolyse (101);
(a") on traite au moins une partie d'un effluent liquide (109) sortant de l'étape (a') dans un réacteur de méthanisation (102) ;
(b) on traite une phase liquide (127) et du biogaz à épurer (110) sortant de l'étape (a") dans une unité de culture de microorganismes phytoplanctoniques (103) ;
(c) on injecte un effluent gazeux (113) contenant du CO₂ dans l'unité de culture de microorganismes phytoplanctoniques ;
(d) on maintient une concentration en NH₃ inférieure à 0,5 g/L dans le réacteur de méthanisation ;
(e) on récupère un biogaz enrichi en méthane au sortir de l'unité de culture de microorganismes phytoplanctoniques.

2. Procédé de fixation de CO₂ et de traitement de déchets organiques selon la revendication 1, dans lequel, pour maintenir une concentration en NH3 inférieure à 0,5 g/L dans le réacteur de méthanisation on procède selon l'étape supplémentaire suivante :
(f) on injecte un effluent gazeux (125) contenant du CO₂ dans le réacteur de méthanisation.

3. Procédé de fixation de CO₂ et de traitement de déchets organiques selon la revendication 1, dans lequel, pour maintenir une concentration en NH3 inférieure à 0,5 g/L dans le réacteur de méthanisation, on maintient un rapport carbone/azote (C/N) moyen dans le réacteur d'hydrolyse entre 10 et 35.

4. Procédé de fixation de CO₂ et de traitement de déchets organiques selon la revendication 3, dans lequel pour maintenir un rapport C/N moyen dans le réacteur d'hydrolyse entre 10 et 35, on module la fraction de déchets organiques introduite dans ledit réacteur d'hydrolyse à l'étape (a').

5. Procédé de fixation de CO₂ et de traitement de déchets organiques selon l'une des revendications 3 à 4, dans lequel pour maintenir un rapport C/N moyen dans le réacteur d'hydrolyse entre 10 et 35, on utilise dans ledit réacteur d'hydrolyse des déchets organiques présentant un rapport C/N supérieur à 25.

6. Procédé de fixation de CO₂ et de traitement de déchets organiques selon la revendication 1, dans lequel, pour maintenir une concentration en NH₃ inférieure à 0,5 g/L dans le réacteur de méthanisation, on maintient un rapport carbone/azote (C/N) moyen dans l'unité de culture de microorganismes phytoplanctoniques entre 10 et 35 ;

7. Procédé de fixation de CO₂ et de traitement de déchets organiques selon la revendication 6, dans lequel pour maintenir un rapport C/N moyen dans l'unité de culture de microorganismes phytoplanctoniques entre 10 et 35, on utilise comme microorganismes pour l'étape (b) des espèces autotrophes ayant un rapport C/N supérieur à 10.

8. Procédé de fixation de CO₂ et de traitement de déchets organiques selon l'une des revendications 6 à 7, dans lequel pour maintenir un rapport C/N moyen dans l'unité de culture de microorganismes phytoplanctoniques entre 10 et 35, on module la quantité d'effluent liquide sortant de l'étape (a") introduite dans l'étape (b), de manière à induire une limitation nutritive propre à modifier la composition des microorganismes de l'unité de culture de microorganismes phytoplanctoniques pour favoriser l'accumulation de lipides et glucides dans lesdits microorganismes.

9. Procédé de fixation de CO₂ et de traitement de déchets organiques selon l'une des revendications 6 à 8, dans lequel pour maintenir un rapport C/N moyen dans l'unité de culture de microorganismes phytoplanctoniques entre 10 et 35, on module le débit entrant du biogaz sortant de l'étape (a") dans l'unité de culture de microorganismes phytoplanctoniques, de manière à contrôler le pH de ladite unité de culture et créer des conditions propres à augmenter le rapport C/N.

10. Procédé de fixation de CO₂ et de traitement de déchets organiques selon l'une des revendications 6 à 9, dans lequel pour maintenir un rapport C/N moyen dans l'unité de culture de microorganismes phytoplanctoniques entre 10 et 35, on module le débit entrant d'effluent gazeux contenant du CO₂ de l'étape (c) dans l'unité de culture de microorganismes, de manière à maintenir un flux de carbone au moins 10 fois supérieur au flux d'azote.

11. Procédé de fixation de CO₂ et de traitement de déchets organiques selon la revendication 10, dans lequel on impose un taux de dilution de la culture de microorganismes phytoplanctoniques inférieur au taux de croissance maximum desdits microorganismes, de manière à induire une limitation par un élément nutritif.

12. Procédé de fixation de CO₂ et de traitement de déchets organiques selon l'une des revendications 1 à 11, dans lequel on utilise dans l'unité de culture de microorganismes phytoplanctoniques des espèces acidophiles ou basophiles pour limiter les contaminations dans ladite unité.

13. Procédé de fixation de CO₂ et de traitement de déchets organiques selon l'une des revendications 1 à 12, comportant l'étape supplémentaire suivante :
(g) on filtre le biogaz enrichi en méthane issu de l'étape (a") dans une colonne d'échange (124) avant l'étape (b).

14. Procédé de fixation de CO₂ et de traitement de déchets organiques selon l'une des revendications 1 à 13, comportant l'étape supplémentaire suivante :
(h) on injecte une fraction (108) de l'effluent liquide sortant de l'étape (a') directement dans l'unité de culture de microorganismes phytoplanctoniques.

15. Système couplé de fixation de CO₂ et de traitement de déchets organiques (100) comportant un réacteur d'hydrolyse/acidogénèse (101) couplé à un réacteur de méthanisation (102), et une unité de culture de microorganismes phytoplanctoniques (103), un premier conduit d'alimentation apte à acheminer un biogaz (110) à épurer depuis le réacteur de méthanisation jusqu'à l'unité de culture de microorganismes phytoplanctoniques, un second conduit d'alimentation apte à acheminer une phase liquide (108, 127) riche en nutriments depuis le réacteur d'hydrolyse et/ou le réacteur de méthanisation jusqu'à l'unité de culture de microorganismes phytoplanctoniques, un troisième conduit d'alimentation apte à acheminer un effluent gazeux (113) extérieur au système et contenant du CO₂ jusqu'à l'unité de culture de microorganismes phytoplanctoniques et un conduit d'évacuation et de récupération de biogaz (114) épuré enrichi en méthane au sortir de à l'unité de culture de microorganismes phytoplanctoniques.

## Patentansprüche

1. Verfahren zur Bindung von CO₂ und zur Verwertung organischer Abfälle durch die Kopplung eines Systems zum anaeroben mikrobiellen Abbau und eines Systems zur Erzeugung von phytoplanktonischen Mikroorganismen, das folgende Phasen umfasst:
(a') die Verarbeitung von Mikroorganismen (105), die aus einer Phytoplanktonkultur stammen, und von organischen Abfällen (104) in einem Hydrolysereaktor (101);
(a") die Verarbeitung von mindestens einem Teil eines flüssigen Abfallprodukts (109) aus Schritt (a') in einem Methanisierungsreaktor (102);
(b) die Verarbeitung einer flüssigen Phase (127) und des zu reinigenden Biogases (110), das aus Schritt (a") stammt, in einer Einheit zur Kultur von phytoplanktonischen Mikroorganismen (103);
(c) die Zuführung eines gasförmigen Abfallprodukts (113), das CO₂ enthält, in die Einheit zur Kultur von phytoplanktonischen Mikroorganismen;
(d) die Aufrechterhaltung einer NH₃ Konzentration von weniger als 0,5 g/l im Methanisierungsreaktor;
(e) der Erhalt von mit Methan angereichertem Biogas am Ausgang der Einheit zur Kultur von phytoplanktonischen Mikroorganismen.

2. Verfahren zur Bindung von CO₂ und zur Verwertung organischer Abfälle nach Anspruch 1, bei dem, um eine NH₃ Konzentration im Methanisierungsreaktor unterhalb von 0,5 g/l zu halten, nach folgendem Zusatzschritt vorgegangen wird:
(f) das Einspritzen eines gasförmigen Abfallprodukts (125), das CO₂ enthält, in den Methanisierungsreaktor.

3. Verfahren zur Bindung von CO₂ und zur Verwertung organischer Abfälle nach Anspruch 1, bei dem, um eine NH₃ Konzentration im Methanisierungsreaktor unterhalb von 0,5 g/l zu halten, das mittlere Verhältnis von Kohlenstoff zu Stickstoff (C/N) im Hydrolysereaktor zwischen 10 und 35 gehalten wird.

4. Verfahren zur Bindung von CO₂ und zur Verwertung organischer Abfälle nach Anspruch 3, bei dem, um das mittlere C/N Verhältnis im Hydrolysereaktor auf einem Wert zwischen 10 und 35 zu halten, der Anteil der organischen Abfälle, die in den in Schritt (a') genannten Hydrolysereaktor gegeben wird, verändert wird.

5. Verfahren zur Bindung von CO₂ und zur Verwertung organischer Abfälle nach einem der Ansprüche 3 bis 4, bei dem, um das mittlere C/N Verhältnis im Hydrolysereaktor auf einem Wert zwischen 10 und 35 zu halten, in besagtem Hydrolysereaktor organische Abfälle verwendet werden, die ein C/N Verhältnis von mehr als 25 aufweisen.

6. Verfahren zur Bindung von CO₂ und zur Verwertung organischer Abfälle nach Anspruch 1, bei dem, um eine NH₃ Konzentration im Methanisierungsreaktor unterhalb von 0,5 g/l zu halten, in der Einheit zur Kultur von phytoplanktonischen Mikroorganismen ein mittleres C/N Verhältnis zwischen 10 und 35 eingehalten wird.

7. Verfahren zur Bindung von CO₂ und zur Verwertung organischer Abfälle nach Anspruch 6, bei dem, um das mittlere C/N Verhältnis in der Einheit zur Kultur von phytoplanktonischen Mikroorganismen auf einem Wert zwischen 10 und 35 zu halten, als Mikroorganismen für den Schritt (b) autotrophe Organismen verwendet werden, die ein C/N Verhältnis von mehr als 10 haben.

8. Verfahren zur Bindung von CO₂ und zur Verwertung organischer Abfälle nach einem der Ansprüche 6 bis 7, bei dem, um das mittlere C/N Verhältnis in der Einheit zur Kultur von phytoplanktonischen Mikroorganismen auf einem Wert zwischen 10 und 35 zu halten, die Menge des flüssigen Abfallprodukts, das aus Schritt (a") stammt und in Schritt (b) eingeleitet wird, in der Weise verändert wird, dass eine Begrenzung über die Nährstoffe erfolgt, was die Zusammensetzung der Mikroorganismen der Einheit zur Kultur von phytoplanktonischen Mikroorganismen verändert, um die Ansammlung von Fetten und Kohlenhydraten in den besagten Mikroorganismen zu fördern.

9. Verfahren zur Bindung von CO₂ und zur Verwertung organischer Abfälle nach einem der Ansprüche 6 bis 8, bei dem, um das mittlere C/N Verhältnis in der Einheit zur Kultur von phytoplanktonischen Mikroorganismen auf einem Wert zwischen 10 und 35 zu halten, die Zuflussmenge in die Einheit zur Kultur von phytoplanktonischen Mikroorganismen von Biogas, das aus Schritt (a") stammt, so variiert wird, dass der pH-Wert der besagten Kultureinheit kontrolliert wird und Bedingungen geschaffen werden, die das C/N Verhältnis erhöhen.

10. Verfahren zur Bindung von CO₂ und zur Verwertung organischer Abfälle nach einem der Ansprüche 6 bis 9, bei dem, um das mittlere C/N Verhältnis in der Einheit zur Kultur von phytoplanktonischen Mikroorganismen auf einem Wert zwischen 10 und 35 zu halten, die Zuflussmenge des gasförmigen, CO₂ enthaltenden Abfallprodukts des Schritts (c), das in die Einheit zur Kultur von Mikroorganismen eingeleitet wird, so variiert wird, dass der Kohlenstofffluss mindestens 10 Mal größer ist, als der Stickstofffluss.

11. Verfahren zur Bindung von CO₂ und zur Verwertung organischer Abfälle nach einem der Ansprüche 10, bei dem ein Verdünnungsgrad der Kultur von phytoplanktonischen Mikroorganismen unterhalb der maximalen Wachstumsrate der genannten Mikroorganismen in der Weise erzwungen wird, dass eine Begrenzung über einen Nährstoff erfolgt.

12. Verfahren zur Bindung von CO₂ und zur Verwertung organischer Abfälle nach einem der Ansprüche 1 bis 11, bei dem in der Einheit zur Kultur von phytoplanktonischen Mikroorganismen acidophile oder basophile Organismen verwendet werden, um die Kontaminierung der genannten Einheit zu begrenzen.

13. Verfahren zur Bindung von CO₂ und zur Verwertung organischer Abfälle nach einem der Ansprüche 1 bis 12, das folgenden Zusatzschritt enthält:
(g) die Filterung des mit Methan angereicherten Biogases des Schritts (a") in einer Austauschsäule (124) vor Schritt (b).

14. Verfahren zur Bindung von CO₂ und zur Verwertung organischer Abfälle nach einem der Ansprüche 1 bis 13, das folgenden Zusatzschritt enthält:
(h) das Einspritzen eines Teils (108) des flüssigen Abfallprodukts aus Schritt (a') direkt in die Einheit zur Kultur von phytoplanktonischen Mikroorganismen.

15. System, das die Bindung von CO₂ und die Verwertung organischer Abfälle (100) miteinander verbindet, das einen Hydrolyse-/ Acidogenesereaktor (101), der mit einem Methanisierungsreaktor (102) gekoppelt ist, und eine Einheit zur Kultur von phytoplanktonischen Mikroorganismen (103), eine erste Zufuhrleitung, die zu reinigendes Biogas (110) vom Methanisierungsreaktor bis zur Einheit zur Kultur von phytoplanktonischen Mikroorganismen transportieren kann, eine zweite Zufuhrleitung, die eine flüssige, nährstoffreiche Phase (108, 127) vom Hydrolysereaktor und/ oder dem Methanisierungsreaktor bis zur Einheit zur Kultur von phytoplanktonischen Mikroorganismen transportieren kann, eine dritte Zufuhrleitung, die ein von außerhalb des Systems stammendes gasförmiges Abfallprodukt (113), das CO₂ enthält, bis zur Einheit zur Kultur von phytoplanktonischen Mikroorganismen transportieren kann, und eine Leitung zur Abfuhr und zum Auffangen von gereinigtem, mit Methan angereichertem Biogas (114) am Ausgang der Einheit zur Kultur von phytoplanktonischen Mikroorganismen umfasst.

## Claims

1. A method for the fixation of CO₂ and for processing organic waste by combining an anaerobic digestion system and a phytoplanktonic microorganism production system, comprising the following steps:
(a') microorganisms (105) originating from a phytoplanktonic culture and organic waste (104) are processed in a hydrolysis reactor (101);
(a") at least part of a liquid effluent (109) coming from step (a') is processed in a methanation reactor (102);
(b) a liquid phase (127) and biogas to be purified (110) coming from step (a") is processed in a phytoplanktonic microorganism culture unit (103);
(c) a CO2-containing gaseous effluent (113) is injected into the phytoplanktonic microorganism culture unit;
(d) a concentration of NH₃ under 0.5g/L is maintained in the methanation reactor;
(e) a methane-enriched biogas is collected when it exits the phytoplanktonic microorganism culture unit.

2. A method for the fixation of CO₂ and for processing organic waste according to claim 1, wherein to maintain a concentration of NH₃ under 0.5g/L in the methanation reactor, the following additional step is used:
(f) a CO2-containing gaseous effluent (125) is injected into the methanation reactor.

3. A method for the fixation of CO₂ and for processing organic waste according to claim 1, wherein to maintain a concentration of NH₃ under 0.5g/L in the methanation reactor, an average carbon/nitrogen (C/N) ratio between 10 and 35 is maintained in the hydrolysis reactor.

4. A method for the fixation of CO₂ and for processing organic waste according to claim 3, wherein to maintain an average C/N ratio between 10 and 35 in the hydrolysis reactor, the fraction of organic waste introduced into said hydrolysis reactor in step (a') is adjusted.

5. A method for the fixation of CO₂ and for processing organic waste according to any of claims 3 to 4, wherein to maintain an average C/N ratio between 10 and 35 in the hydrolysis reactor, organic waste having a C/N relationship over 25 is used in said hydrolysis reactor.

6. A method for the fixation of CO₂ and for processing organic waste according to claim 1, wherein to maintain a concentration of NH₃ under 0.5g/L in the methanation reactor, an average carbon/nitrogen (C/N) ratio between 10 and 35 is maintained in the phytoplanktonic microorganism culture unit.

7. A method for the fixation of CO₂ and for processing organic waste according to claim 6, wherein to maintain an average C/N ratio between 10 and 35 in the phytoplanktonic microorganism culture unit, autotrophic species having a C/N ratio over 10 are used as microorganisms for step (b).

8. A method for the fixation of CO₂ and for processing organic waste according to any of claims 6 to 7, wherein to maintain an average C/N ratio between 10 and 35 in the phytoplanktonic microorganism culture unit, the quantity of liquid effluent exiting step (a") introduced in step (b) is adjusted, so as to induce a nutrient limitation that is able to modify the composition of the microorganisms in the phytoplanktonic microorganism culture unit to favour the accumulation of lipids and carbohydrates in said microorganisms.

9. A method for the fixation of CO₂ and for processing organic waste according to any of claims 6 to 8, wherein to maintain an average C/N ratio between 10 and 35 in the phytoplanktonic microorganism culture unit, the intake flow rate of the biogas exiting step (a") into the phytoplanktonic microorganism culture unit is adjusted, so as to control the pH of said culturie unit and create proper conditions for increasing the C/N ratio.

10. A method for the fixation of CO₂ and for processing organic waste according to any of claims 6 to 9, wherein to maintain an average C/N ratio between 10 and 35 in the phytoplanktonic microorganism culture unit, the intake flow rate of the CO₂-containing gaseous effluent of step (c) into the microorganism culture unit is adjusted, so as to maintain a carbon flow at least 10 times higher than the nitrogen flow.

11. A method for the fixation of CO₂ and for processing organic waste according to claim 10, wherein a dilution rate of the phytoplanktonic microorganism culture less than the maximum growth rate of the said microorganisms is established, so as to induce a nutrient limitation.

12. A method for the fixation of CO₂ and for processing organic waste according to any of claims 1 to 11, wherein acidophilic or basophilic species are used in the phytoplanktonic microorganisms culture unit, in order to limit contaminations in said unit.

13. A method for the fixation of CO₂ and for processing organic waste according to any of claims 1 to 12, comprising the following additional step:
(g) the biogas enriched in methane exiting step (a") in filtered on an exchange column (124) before step (b).

14. A method for the fixation of CO₂ and for processing organic waste according to any of claims 1 to 13, comprising the following additional step:
(h) a fraction (108) of liquid effluent exiting step (a') is injected directly into the phytoplanktonic microorganism culture unit.

15. A combined system for the fixation of CO₂ and for processing organic waste (100) comprising a hydrolysis/acidogenesis reactor (101) coupled with a methanation reactor (102), and a phytoplanktonic microorganism culture unit (103), a first supply pipe for carrying a biogas (110) to be purified from the methanation reactor to the phytoplanktonic microorganism culture unit, a second supply pipe for carrying a liquid phase (108, 127) rich in nutrients from the hydrolysis reactor and/or the methanation reactor to the phytoplanktonic microorganism culture unit, a third supply pipe for carrying a CO₂-containing gaseous effluent (113) from outside the system to the phytoplanktonic microorganism culture unit and a pipe for discharging and recovering the methane-enriched purified biogas (114) exiting the phytoplanktonic microorganism culture unit.
